# EUROPEAN PATENT SPECIFICATION

(11) **EP 1 683 518 B1**
(45) Date of publication and mention of the grant of the patent: **22.04.2009**
(21) Application number: 05425357.0
(22) Date of filing: 23.05.2005
(51) Int. Cl.: A61K 31/167, A61K 47/10, A61K 9/08

(54) **Pharmaceutical formulation of formoterol and propylene glycol**
Pharmazeutische Zusammensetzung enthaltend Formoterol und Propylenglycol
Formulation pharmaceutique contenant du formoterol et du propylen glycol

(30) Priority: 19.01.2005 IT RM20050022
(43) Date of publication of application: 26.07.2006
(73) Proprietor: Italchimici SpA, 00040 Pomezia (IT)
(72) Inventor: Sonaglia, Achille, 00040 Pomezia (IT); Annaloro, Raffaele, 00040 Pomezia (IT)
(74) Representative: Conforti Cioncoloni, Marisa

(56) References cited:
- EP-A- 0 943 330
- EP-A- 1 302 200
- US-A- 6 150 418
- US-A1- 2005 009 923

## Description

The present invention relates to a pharmaceutical formulation of a stable Formoterol-containing propylene glycol solution, without addition of preservatives, for use in inhalation therapy. Such a solution can be manufactured in the form of single-dose glass ampoule so that a simple, ready administration to a patient by means of electric nebulisers, which are commonly available on the market, after mixing said solution with distilled water, purified water or saline solution, is permitted.

Formoterol is a selective agonist of pulmonary beta2-adrenergic receptors. It is endowed with a quick onset of action, strong bronchodilation activity, whose effect lasts for up to 12 hours from its administration. The inhalation administration is the preferred one, as it allows the active substance to reach directly the site of action permits the use of lower daily dosages and causes fewer systemic side effects as compared to the systemic administration.

Formoterol is commonly used in bronchial asthma therapy in the form of powder for inhalation of metered dose inhaler (MDI), and the latter formulation needs to be stored at low temperatures due its high instability.

Patents to Boehringer Ingelheim Pharma (e.g., US-6,150,418 granted on November 21, 2000 and EA3960 published on October 30, 2003) already is concentrated in these solutions and suspensions (between about 75 mg/ml and about 500 mg/ml).

However such patents do not teach how to preserve dilute Formoterol, i.e. in the necessary form to be ready to be mixed with water and used.

An object of the present invention is the preparation of a single-dose ampoule containing Formoterol as an active substance in a stable solution, that even without preservatives will maintain an insignificant bioburden for a commercially viable period of time till the opening of the ampoule for administration to the patients.

Another object of the invention is to allow a preparation containing Formoterol as an active substance to be delivered very efficiently via an electric nebuliser directly to the target organ, i.e. the lungs.

Therefore, this invention relates to a pharmaceutical formulation of a formoterol-containing propylene glycol solution for use in inhalation therapy according to enclosed Claim 1.

A Formoterol-containing propylene glycol solution can be prepared by a method comprising the following steps:
- dissolving Formoterol in a suitable reactor in a required amount of propylene glycol by stirring, in order to obtain a concentrated propylene glycol solution;
- diluting the concentrated propylene glycol solution to volume with propylene glycol to obtain the final solution;
- filtering the final solution; and
- filling ampoules with the final solution to a desired volume.

An advantage of the invention is that the solution obtained remains unalterated both in terms of stability of active ingredient and bioburden for a period of time that makes it interesting for commercial use.

The solution contained in an ampoule, usually 1 ml, will be mixed with 1-3 ml of distilled water for injections or purified water or saline solution into the nebulising chamber of an electro-mechanical nebuliser immediately before the administration to the patient.

A method of preparing a Formoterol-containing water-propylene glycol solution according to the present invention is explained below.

In a suitable reactor, such an amount of Formoterol, so that a concentration variable between 0.001 and 0,06 per cent, for example 0,006 per cent, will be obtained in a final solution, is dissolved in the required amount of propylene glycol by stirring, in order to obtain a concentrated propylene glycol solution.

The concentrated propylene glycol solution is diluted to volume to obtain the finished solution of required concentration.

The finished solution is filtered through a 0,22 µm wide mesh filter and then is packaged into single-dose 1 ml ampoules made of amber-coloured, neutral glass or any other suitable similar primary container such as polyethylene or polypropylene ampoules.

Stability studies carried out with inventors' laboratories according to the ICH (International Conference on Harmonisation) guidelines, showed that a typical Formoterol fumarate-containing propylene glycol solution at a concentration of 60 micrograms per ml, in ampoules made of amber glass, remains unaltered at 5° Celsius for a period up to 12 months. Once the ampoules are brought to room temperature, the solution will remain stable for a further period of 7 months if stored at that temperature. Yet, the tolerability of the inhalation solution appeared good, and the respirable portion was in the range of 10-30%.

## Claims

1. A pharmaceutical formulation of a Formaterol-containing propylene glycol solution for use in inhalation therapy, **characterised by** the following composition:
- Formoterol, as an active principle, in a concentration variable between 0.001 and 0.06 per cent weight/volume;
- a solvent of propylene glycol and water in the required amount to achieve 100 per cent of a finished solution.

## Patentansprüche

1. Une formule pharmaceutique d'une solution de propylène glycol contenant du Formotérol, destinée aux thérapies par inhalation et **caractérisée par** la composition suivante :
Formotérol, en tant que substance active dans des concentrations variables comprises entre 0,001 et 0,06 % et un solvant composé de propylène glycol et d'eau dans la quantité requise pour obtenir 100 % d'une solution finie.

## Revendications

1. Eine pharmazeutische Rezeptur einer Formoterol-haltigen Propylenglykol-Lösung zur Anwendung in der Inhalationstherapie, **gekennzeichnet durch** die folgende Zusammensetzung:
Formoterol als Wirkstoff in einer Konzentration zwischen 0,001 und 0,06 Prozent, und ein Lösungsmittel aus Propylenglykol und Wasser in der erforderlichen Menge, um 100 Prozent fertige Lösung zu erhalten.
